# EUROPEAN PATENT SPECIFICATION

(11) **EP 3 589 205 B1**
(45) Date of publication and mention of the grant of the patent: **03.02.2021**
(21) Application number: 18710961.6
(22) Date of filing: 27.02.2018
(51) Int. Cl.: A61B 5/15, A61B 5/154

(54) **SAMPLE TUBE HOLDER AND SYSTEM**
PROBENRÖHRCHENHALTER SOWIE SYSTEM
SUPPORT DE TUBE D'ÉCHANTILLON ET SYSTÈME

(30) Priority: 01.03.2017 US 201762465563 P; 30.05.2017 US 201762512530 P
(43) Date of publication of application: 08.01.2020
(73) Proprietor: Fenwal, Inc., Lake Zurich, IL 60047 (US)
(72) Inventor: LYNN, Daniel, Lake Zurich, IL 60047 (US); KOEHLER, Lynn, Lake Zurich, IL 60047 (US); MATSER, Timo, Lake Zurich, IL 60047 (US)
(74) Representative: Maikowski & Ninnemann Patentanwälte Partnerschaft mbB
(86) International application number: PCT/US2018/019840
(87) International publication number: WO 2018/160523

(56) References cited:
- EP-A1- 2 450 070
- WO-A1-2016/152950
- DE-U1- 20 108 230
- JP-U- 3 123 813
- JP-U- 3 137 417

## Description

### Field of the Disclosure

This disclosure relates to a sample tube holder of the general type commonly associated with medical fluid flow circuits for receiving sample tubes or vials, such as Vacutainer ® blood collection tubes or vials, for collecting fluid samples for laboratory testing or evaluation.

More specifically, the present disclosure concerns a sample tube holder that may be provided individually or as an associated part of a medical fluid flow circuit of the type that may include a vascular needle and connected plastic tubing for collecting or processing blood or blood components. For example, it is common to collect whole blood from patients for routine testing or from healthy donors for processing to obtain blood components that may be administered to patients in need of such a blood component as part of a therapeutic procedure. Typically, such a fluid flow circuit includes a donor or patient access device, such as a needle, tubing connecting the needle to downstream collection containers or processing devices, such as a centrifuge chamber that separate the blood into component parts, and other associated devices or containers such as filters for removing leukocytes, containers of anticoagulant and/or blood component preservatives and the like.

The use of needles for drawing blood from a donor or patient has a known risk of post-procedure accidental needle stick for the professional carrying out the particular procedure. Certain precautions have been mandated for covering needles after use to help reduce the risk of such needle sticks, and it is common for access needles to have an associated needle protector that is activated or placed over the needle after use so as to protect against accidental sticks. For example, WO 2016/152950 A1 refers to a blood collection set equipped with a collection needle and a holder in form of a hollow, cylindrical holder body having an insertion port and a lid capable of opening and closing the insertion port.

In addition to the needles that are used to access a patient's or donor's blood vessel, the sample tube holder that is commonly part of such blood collection systems also has an internal needle. The sample tube holder is typically a cylindrical or barrel shaped device that is generally closed at one end, with a sampling needle projecting internally into the barrel from the closed end. The internal sampling needle is usually connected to a port in the closed end that leads directly or indirectly to an access needle or to tubing that leads to an access needle. When it is desired to take a sample, an empty sample tube or vial, typically with an internal vacuum and a puncturable end septum or seal, is inserted into the sample tube holder until the internal sampling needle pierces the septum. The vacuum in the sample container or vial assists in drawing blood into the sample tube through the internal sampling needle from the access needle or the associated plastic tubing through which blood or blood components flow. Sample tube holders of various types and designs may be found in one or more of the following patents: U.S. Patents Nos. 4,932,418; 5,030,209; 5,752,936; 6,540,696; 7,435,231; and 7,479,131.

It may be further desirable to protect or guard the internal sampling needle of the sample tube holder from accidental needle stick, such as from a user or other person sticking their finger into the sample tube holder. One design to help prevent such accidental sticks may be found in U.S. Patent No. 6,540,696. It discloses inserting the vascular access needle and needle protector into the open end of the sample tube holder so that the internal needle of the sample tube holder extends into the needle protector. This design calls for particular retaining means to be employed in the sample tube holder to engage the needle protector and help hold the needle protector in the sample tube holder.

There continues to be a desire for further development of sample tube holders with protective features and the present disclosure is directed to such a sample tube holder, the blood collection or processing system employing such sample tube holder and the method of using it. One non-limiting embodiment is described below for purposes of illustration and disclosure and not for purposes of limitation. Other designs and configurations may incorporate the features of the present disclosure, and the scope of the invention is as defined in the claims now or hereafter filed.

In accordance with one non-limiting aspect of the present subject matter, a sample tube holder may be provided including a body, open at one end, and including a lid pivotally attached to the body and movable to a closed position at least partially closing the open end, the lid including an aperture configured to capture an access needle and needle protector within the body when the lid is in the closed position and a latch to retain the lid in a closed position.

The sample tube holder aperture may be sized to capture an access needle and needle protector within the body. The aperture may extend through at least one edge of the lid. More particularly, the sample tube holder lid may be pivotally attached to the body at one side edge and the aperture may extend through a side edge opposite the one side edge. Also, the access needle and needle protector may be part of a needle assembly, and the aperture configured to allow a portion of the needle assembly to extend through the aperture while capturing or confining the access needle and needle protector within the body.

A portion of sample tube holder lid bordering the aperture may be raised or elevated above the plane of the rest of the lid or the body open end to provide enlarged internal dimension for receiving, for example, access needle assemblies of differing length. More specifically, the portion of the lid adjacent the entire edge of the aperture may be raised or elevated above the plane of the lid or body open end to provide greater internal space.

The sample tube holder lid may be pivotally attached to the body by a hinge comprising a passive connecting hinge portion and an active hinge portion. For example, the active hinge portion may be configured or biased to hold the lid in an open and/or closed position.

To retain the lid in the closed position, the sample tube holder may comprise a flange at the open end and one of the flange and the lid may include a latching member and the other of the flange and the lid include a latching member engagement. The latching member may comprise a hook and the latching member engagement comprise a hook receiver. At least two latching members may be provided on the lid and the flange may comprise at least two latching member engagements to securely hold the lid in a closed position and prevent accidental opening.

The sample tube holder may include an internal sampling needle within the body and extending from an end of the body opposite the open end. Alternatively, the sample tube holder may be configured to allow user attachment of a sampling needle, for example, via threaded, snap-fit or press-fit arrangement.

The method of using the sample tube holder of claim includes inserting an access needle and needle protector into the open end of the sample tube holder and closing and latching the lid, thereby capturing the access needle and needle protector in the sample tube holder. The presence of the access needle and needle protector in the sample tube holder body helps prevent accidental insertion of a finger into the tube holder body and accidental contact/stick with any sampling needle located in the body.

The sample tube holder as described herein may be provided separately or as part of a medical fluid flow system including an access needle for accessing the vascular system of a human subject, a needle protector for protecting the access needle from accidental stick after usage, and a sample tube including a body, open at one end, a lid pivotally attached to the body and movable to a closed position at least partially closing the open end, and a fluid port at an end of the body opposite the open end, the port being configured to allow fluid flow connection with the medical fluid flow system, with the lid including an aperture configured to capture the access needle and needle protector within the body when the lid is in the closed position and a latch to retain the lid in a closed position.

Such a medical fluid flow system may include a sample tube holder in which the lid aperture is sized to capture or confine the access needle and needle protector within the body. The aperture may extend through at least one edge of the lid, and more particularly, the lid may be pivotally attached to the body at one side edge of the lid and the aperture may extend through a lid side edge opposite the one side edge where the hinge is located. The aperture may be configured to allow a portion of the needle assembly to extend through the aperture while capturing the access needle and needle protector within the body. The medical fluid flow system sample tube holder may include an internal sampling needle within the body and extending from an end of the body opposite the open end, which is protected against accidental needle sticks when the access needle and needle protector are inserted into the body.

In the medical fluid flow system, a portion of sample tube holder lid bordering the aperture may be elevated or raised above the plane of the rest of the lid or the plane of the body open end to provide greater distance between a closed lid and the closed end of the body to accommodate needle assemblies of differing length. More particularly, the portion of the lid adjacent to the entire edge of the aperture may elevated or raised above the plane of the body open end to provide greater interior dimension. In other words, the sample tube holder, whether by itself or as part of a medical fluid flow system, may have a lid aperture that includes a peripheral edge of relatively raised dimension to provide additional interior space and axial length between an end of the body opposite the open end and at least a portion of the lid when the lid is in the closed position.

The lid of the sample tube holder in the medical fluid flow system may be pivotally attached to the tube holder body by a hinge comprising a passive hinge portion and an active hinge portion. The active hinge portion may be configured to bias the lid to an open and/or closed position.

To hold the lid closed after the access needle and needle protector are inserted, the medical fluid flow system sample tube holder body may comprise a flange at the open end and one of the flange and the lid may including a latching member, and the other of the flange and the lid may include a latching member engagement. More specifically, the latching member may comprise a hook and the engagement may comprise a hook receiver or aperture. The lid may further comprise at least two latching members and the flange comprise at least two latching member engagements to provide more secure connection of the lid in the closed position to avoid accidental opening.

### Brief Description of The Drawings

Fig. 1 is an exploded plan view, partially in section of a vein or vascular system access needle assembly, including a pre-use needle cover and post-use needle protector.
Fig. 2A is a perspective view of the assembled needle assembly of Fig. 1 before use.
Fig. 2B is a perspective view of the needle assembly of Fig. 2A with the needle cover removed, e.g. to prepare the access needle for use.
Fig. 2C is a perspective view of the needle assembly of Fig. 2B as it would appear after use, with the needle protector or shield advanced to a position covering the access needle to help prevent accidental sticks.
Fig. 3 is a perspective view of one embodiment a sample tube holder employing various aspects of this disclosure, as explained more fully below. This view shows the sample tube holder with the lid or cover in a fully open position.
Fig. 4 is a perspective view of the sample tube holder of Fig. 3 taken from a different angle and with the lid or cover closed.
Fig. 5A is a cross-sectional view of the sample tube holder of Fig. 3 with the lid or cover closed.
Fig. 5B is a top view of the sample tube holder of Fig. 5A.
Fig. 5C is a top view of the sample tube holder similar to Fig. 5B, but with the lid or cover fully open.
Fig. 5D is an enlarged cross sectional view of the upper part of the sample tube holder of Fig. 5C, taken along line D-D of Fig. 5C, with the lid or cover fully open.
Fig. 5E is an enlarged cross sectional view taken along line E-E of Fig. 5B, showing a latching arrangement for holding the lid or cover closed.
Fig. 5F is an enlarged cross-sectional view taken along line F-F of Fig. 5A.
Fig. 6A is a view of the underside of an alternative configuration of a sample tube lid or cover that may be used in the sample tube holder of Fig. 3 and having a raised or elevated portion.
Fig. 6B is a top view of the alternative lid or cover of Fig. 6A.
Fig. 6C is a partial side view of a sample tube holder employing the alternative lid or cover of Figs. 6A and 6B.
Fig. 7 is an enlarged partial sectional view of the alternative cover or lid of Fig. 5.
Figs. 8-10 illustrate a sequence of inserting a used access needle and needle protector of Figs. 1-2 into the sample tube holder of Figs. 3-6.

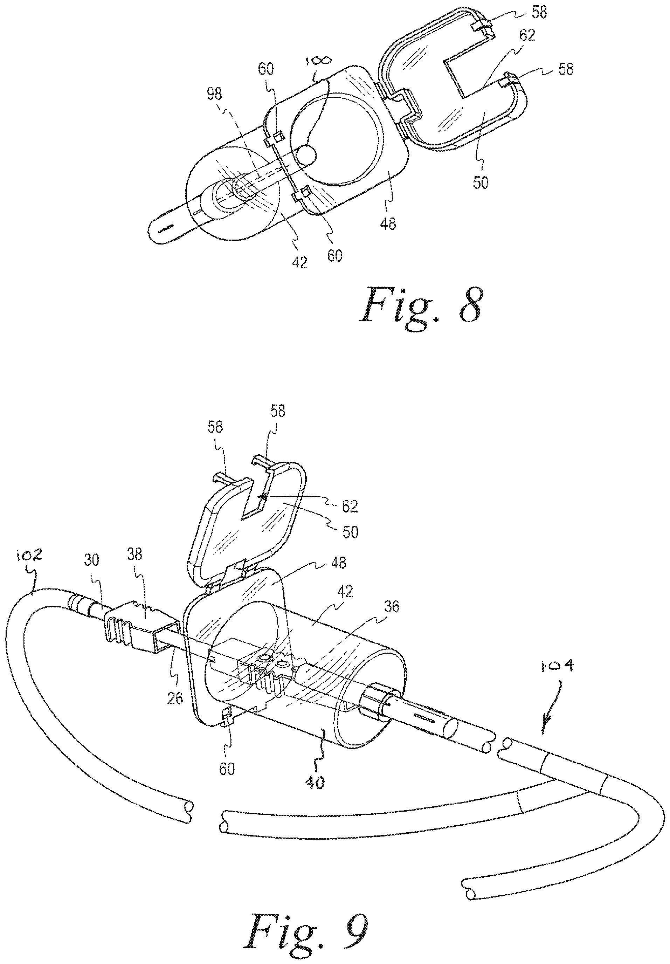

Fig. 8 is a perspective view of the sample tube holder with the cover or lid open and ready to receive a used vascular access needle assembly.
Fig. 9 is a perspective view illustrating insertion of a used access needle, with needle protector in the protecting position over the needle, into the open end of the tube holder.

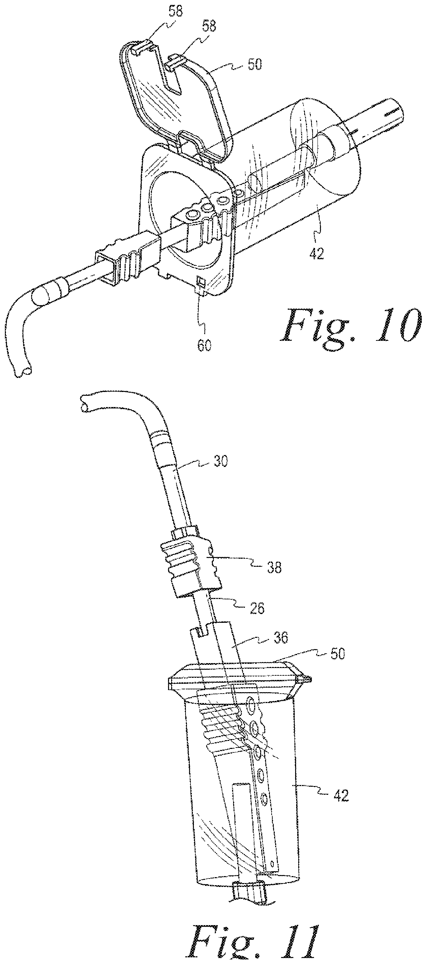

Fig. 10 shows further insertion of the access needle assembly into the sample tube holder.
Fig. 11 shows the sample tube holder with the cover or lid closed and capturing or confining the used access needle assembly in the sample tube holder for disposal.

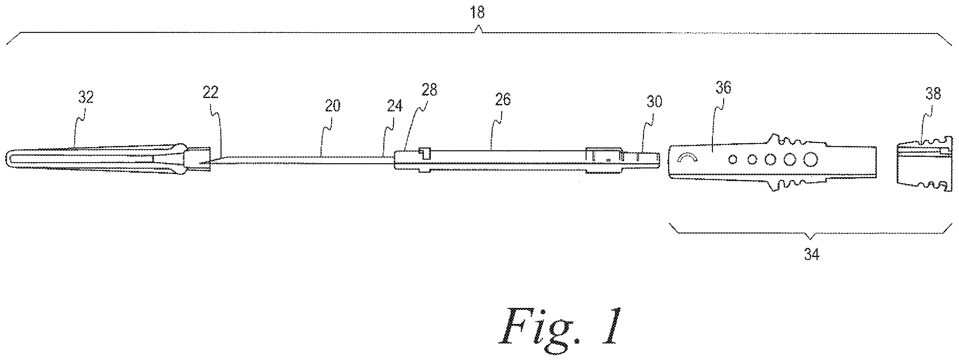

Turning now to a more detailed description of the drawings, Fig. 1 illustrates one example of a vascular access needle arrangement or assembly, generally at 18, that may be employed in a blood collection or sampling fluid flow circuit and may be used with the sample tube holder of the present disclosure for post-use protection against accidental sticks. The needle assembly shown in Fig. 1 is solely for purpose of illustration, as other needle assemblies of varying design may also be used.

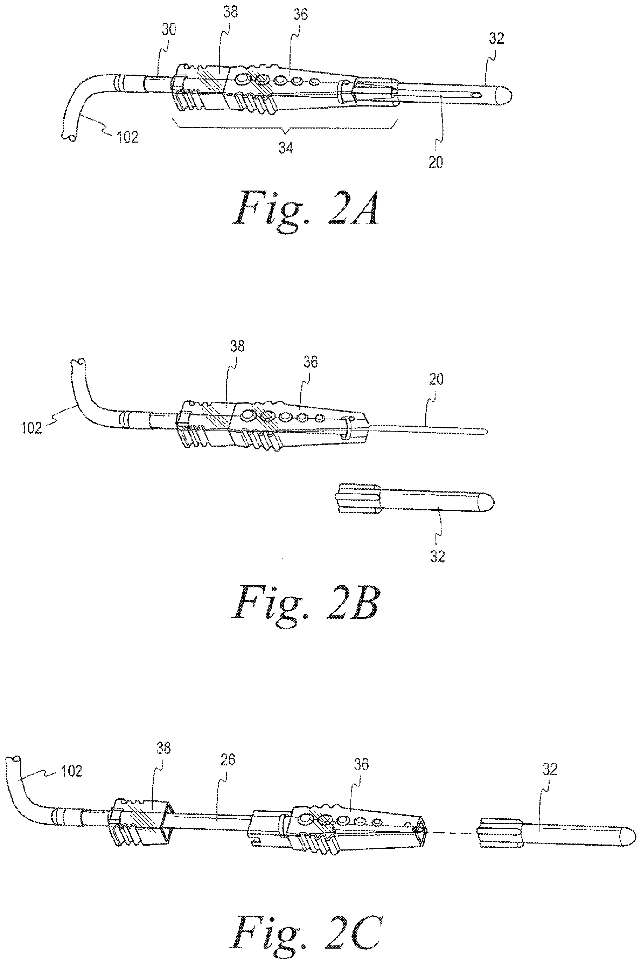

As shown in Fig. 1, the needle assembly 18 includes an access needle 20 of stainless steel or other suitable material, pointed at the distal end 22 for transcutaneous insertion, and having an internal fluid flow passageway extending from the distal end to proximal end 24; an elongated needle holder or base 26 fixedly attached at its distal end 28 to the proximal end of needle 20. The elongated needle base 26 has an internal fluid flow path for flow of blood or other fluid from the access needle to tubing 102 or to a blood collection or sampling system 104 (see, e.g., Figs. 2A-2C and 9) connected to the other or proximal end 30 of the needle base.

For pre-use needle protection, an elongated removable cover 32 is positioned over the needle for temporary stick protection until the needle must be used. The needle cover may be temporarily attached to and removable from the needle base, e.g., frangibly or frictionally connected to the needle base 26, allowing removal when the needle must be used.

For post-use protection against sticks, a needle protector or protective shield 34 is mounted on the base and configured such that an elongated portion 36 of the needle protector can be advanced by sliding it along the base to a position over the access needle after the procedure is complete. When so advanced, the elongated portion 36 extends over the sharpened distal end 22 of the access needle 20 to protect against accidental needle sticks. The hub portion 38 of the needle protector remains proximate to the proximal end 30 of the needle holder when the elongated portion is advanced distally to cover the needle 20.

Figs 2A-2C illustrate the needle assembly of Fig. 1 in different operative positions or states. Fig. 2A shows the needle assembly before use, with the removable needle cover 32 covering the needle 20, the needle protector 34 covering most of the needle base 26, and the proximal end 30 of needle base 26 protruding from the hub portion 38 of the needle protector 34, which may be connected as part of a medical fluid flow system such as system 104.

Fig. 2B shows the needle cover removed from the access needle 20, as it would be in preparation for use of the needle. In Fig. 2c, the post-use position, the needle protector portion 36 is slid into position over the access needle and covering the pointed distal end to protect against post-use accidental sticks.

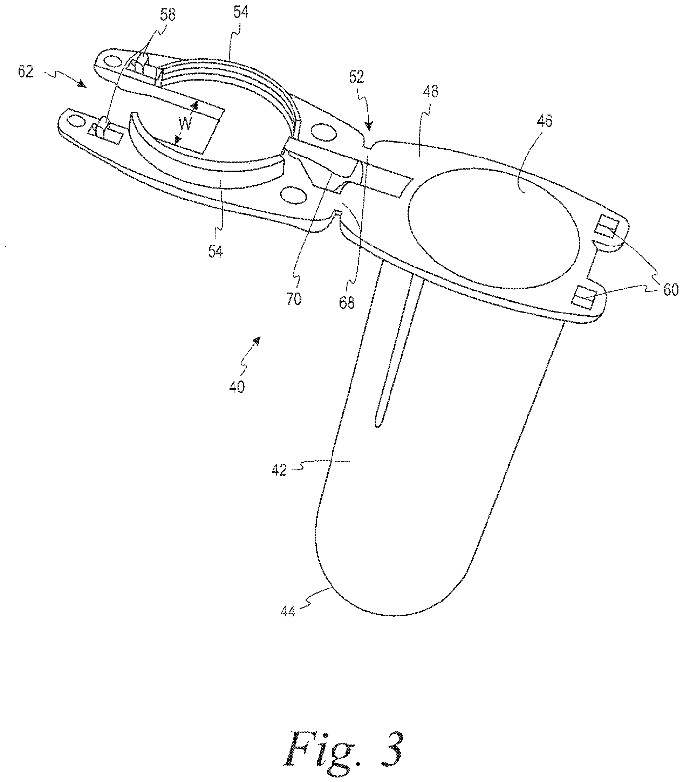

Fig. 3 is a perspective view of one embodiment of a sample tube holder, generally at 40, in accordance with the present disclosure. The sample tube holder includes an elongated cylindrical or barrel shaped body 42 substantially closed at one end 44 and open at the other end 46. The body 42 may optionally be referred to as the barrel. The open or proximal end of the body has a flange 48 that is pivotally attached to a lid or cover 50, such as by hinge structure 52 that allows movement of the lid between an open position (allowing sample vial insertion) and a closed position extending over the open end of the body.

The lid 50 may optionally have one or more arcuate segments 54, which correspond approximately in size or diameter to the curved inner cylindrical surface of the body 42 projecting from the underside of the lid for generally close-fitting or conforming and guiding receipt into the open end of the body when the lid is closed over the open end of the body.

A latch arrangement of any suitable configuration may also be provided for holding the lid closed. As illustrated, for example, a latch may be integrally molded as part of the lid 50 and body 42. More specifically, one of the lid 50 or the body flange 48 may include one or more latching members, such as extending hooks 58, and the other of the lid or the flange may include one or more latching member engagements, such as mating openings 60. In the illustrated embodiment, the hooks 58 project from the underside of the lid 50 and, when the lid is closed, extend into a hook-receiving openings 60 located in flange 48, opposite the hinge 40, providing a secure double locking arrangement. Other latching arrangements providing similarly secure latching may also be used.

One or more and, for example, all of the body 42, lid or cover 50, hinge structure 52, and latch arrangement 56 may be integrally and economically molded as one unitary piece from any suitable plastic material, such as polypropylene or polystyrene. As illustrated, the body, lid, hinge structure, arcuate segments 54 and latch hooks 58 and mating openings 60 are all integrally molded as one unitary piece.

The lid 50 also has an aperture 62 provided therein. The aperture allows the lid to be closed after a used access needle assembly is inserted into the body 42. The illustrated aperture 62 extends or opens through the outer perimeter or edge of the lid. More particularly, the illustrated aperture 62 opens through the edge of the lid opposite the hinge structure 52 for ease of closing over an inserted needle assembly, although the aperture could also be formed so as to extend through the perimeter or edge of the lid at any suitable side or end location that allows closing of the lid after a used needle assembly has been inserted into the body. As may be visualized, and as illustrated later, when a used needle assemble is inserted into the body, a portion of it, such as the a proximal portion 30 of the needle base 26, hub portion 38 and a segment of connected fluid flow tubing 102, may still protrude from the open end of the body, and the aperture in the lid allows the lid to close and for the hub portion and/or connected tubing to extend through the aperture.

The aperture 62 may be formed in the lid 50 in any suitable manner, such as during initial molding or by cutting. In the illustrated arrangement, the aperture is in the form of a substantially rectangular notch that extends from one edge of the lid toward the hinge structure 52.

The aperture is sized so that the closed lid 50 captures or traps the used access needle 20 and associated needle protector portion 36 within the body 42, thus adding further protection to help avoid accidental needle sticks as well as to reduce the potential for blood spillage from the access needle. More specifically, the aperture is configured so that when the lid is closed it prevents accidental withdrawal of the used needle assembly from the body 42 through the aperture. For example, the aperture is dimensioned so as to be smaller than the portion of the needle assembly (i.e, the combined access needle and needle protector portion) located within the body, so that it cannot be pulled through the aperture when the lid is closed. As illustrated, the width W of the slot in the lid may be narrower than the proximal end of the needle protector portion 36 that is located in the body. Thus, when the lid 50 is closed over a used needle assembly 18 and latched, the used needle assembly is trapped within the sample tube holder body 42. It should be noted that the needle assembly may also be described herein as captured or confined in the tube holder body, and no difference is intended. When so trapped, captured or confined, the used needle assembly is protected against accidental or inadvertent removal from the body of the sample tube holder and the infusion needle 20 is afforded another barrier against accidental needle sticks. In other words, when a used needle assembly 18 is inserted into the body 42 of the sample tube holder 40 and the lid 50 closed thereover and latched, the aperture 62 allows the proximal end of the needle assembly to extend from the sample tube holder body through the lid, thus allowing the lid to close, while the access needle and needle protector are captured within the sample tube holder body and cannot be withdrawn through the aperture due to the smaller size of the aperture as compared to the needle protector 36. Thus, no special retention means needs to be provided within the sample tube holder body to directly engage or retain the needle protector - it is trapped within the sample tube holder body by the closed lid.

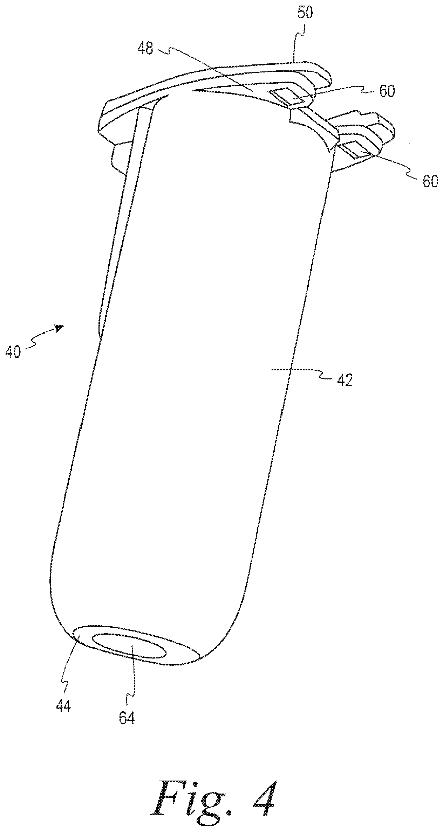

As earlier described, sample tube holders of the type involved here typically have an internal needle that is mounted at the generally closed end of the body 42 and extends upwardly within the holder from the generally closed end toward the open end. This sampling needle may be located within a flexible dust cover that is displaced when a sample vial is inserted into the body 42. This internal needle is connected through a port 64 (Fig. 4) in the closed end of the body to tubing or other apparatus that connects to the fluid source that is to be sampled. The internal needle may be provided at the time the tube holder is manufactured or, alternatively, the sample tube holder body may have a port that employs a threaded connection, snap-fit or press fit connection, with respect to which an internal or sampling needle may be assembled. The port 64 may also extend from the body 42 in the shape of a male luer or luer-lock fitting for connection to other medical devices or components.

Typically, but not exclusively, the port 64 may be connected to tubing 102 that is part of a medical fluid flow system and leads directly or indirectly to a vascular access needle assembly such as 18. When the user wishes to remove a sample of blood or other fluid, a vacuum-containing vial (sealed at one end by a puncturable seal) is inserted into the body until the internal needle punctures the seal and the vacuum in the vial assists in drawing blood or other fluid into the vial. After the sample is taken, the vial may be removed from the sample tube holder body. Although the internal sampling needle of the sample tube holder is recessed in the body 42, when the used needle assembly 18 (with the needle protector over the access needle 20) is inserted into the body, such as along side or next to the internal needle, the physical presence of the used needle assembly in the tube holder body helps prevent someone from inserting their finger into the body sufficiently far to contact the sampling needle. When the lid 50 is closed and latched over a used needle assembly 18, the internal sampling needle in the sample barrel or body 42 and the access needle are both further protected against someone accidentally or intentionally making contact with the needles.

Figure 4 shows the sample tube holder 40 of Figure 3 from a different perspective, with the lid 50 closed and latched. In this view the exemplary port 64 may be better seen at the generally closed end of the sample tube holder body 42.

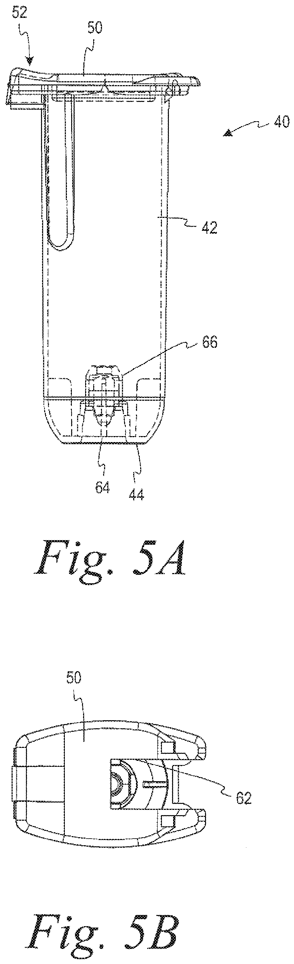

Figs. 5A-5F show additional details of the sample tube holder 40 of Fig. 3. Fig. 5A is a side elevational view of sample tube holder 40, partially in section, with the lid 50 in a closed and latched position. As seen in this version, the distal or generally closed end of the sample tube holder body 42 has the port 64 communicating with a needle mount 66 that is configured, such as by threads, to allow for mounting of an internal needle within the body 42.

Fig. 5B is a top view of Fig. 5A, looking down onto the closed lid 50. In that figure, the lid aperture 62 is readily visible, as is a portion of the internal needle mount 66 located at distal closed end of the body 42.

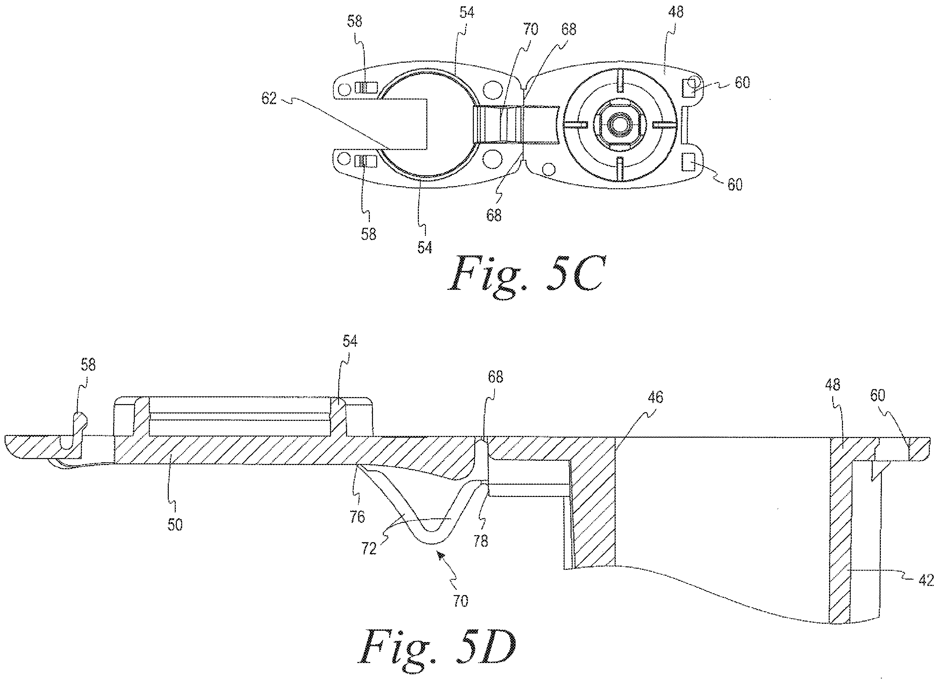

Fig. 5C is a top view of Fig. 5A, but with the lid 50 in a fully open position. This figure allows for better viewing of the underside of lid 50, the hinge structure 52, arcuate segments 54 and latch hooks 58. Similarly, the flange 48 located at the proximal or open end of tube holder body 42 is plainly visible with the openings 60 for receiving the latch hooks of lid 50 when the lid is closed.

Fig. 5D is an enlarged cross-sectional view of the lid 50 and the portion of the tube holder body 42 to which it is attached, in the fully open position, taken along line D-D of Fig. 5C. This enlarged view illustrates the projection of arcuate segment 54 from the underside of lid 50 and also shows one of the openings 60 for a latch hook. Also, various other features of the illustrated hinge structure 52 are visible in this figure. Specifically, one or more connecting flexible hinge portions 68 extend between the flange 48 and lid 50 and may be considered to be relatively passive in that they allow the lid to freely pivot about the axis of the flexible hinge portions. In the illustrated hinge structure, there are two flexible hinge portions 68 and, as shown, they flank an active hinge element that is configured to provide a biased hinge type function that tends to hold the lid 50 open and/or closed. This illustrated active hinge element includes an angled member 70, with two angled legs 72 joined at a vertex 74. The resilience of the plastic material of which the is made and the location of the connection points 76, 78 where the ends of the legs are attached, respectively, to the lid 50 and the body 42 tend to provide a biasing action or force. The connection point 78 with the body 40 is below or offset from the plane of the hinge portions 68, which requires that the angled member 70 flex at the vertex from its normal angular position as the lid opens or closes. This creates a bias, due to the natural resilience of the angled member material, that tends to hold the lid either open or closed depending, respectively, on whether the lid is located in either a more open position or a more closed position relative to an angular position of inflection of the lid where the vertex flexing is greatest.

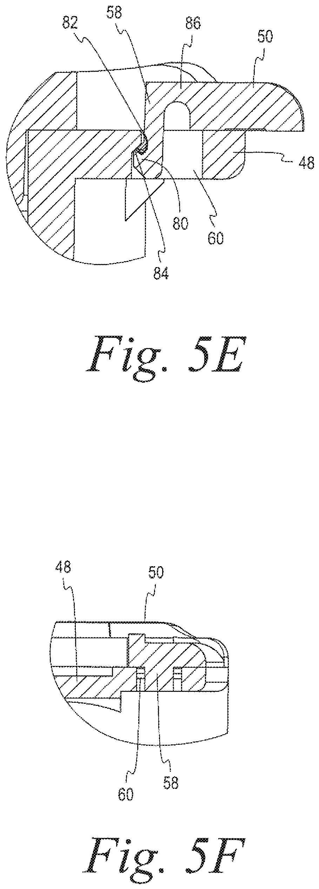

Figure 5E is an enlarged cross-sectional view, taken along line E-E of Fig. 5B, showing the lid latching engagement between at least one of the hooks 58 located on lid 50 and one of the hook openings 60 located in flange 48. In the illustrated embodiment each hook is of identical design, as is each of the hook openings, although they could differ if desired. The double locking feature provided by two hooks better provides robust resistance to accidental or unintended opening of the lid. As can be seen in Fig. 5E, hook 58 has an enlarged end portion 80 with an inclined surface 82. Due to resilience of the hook material, the inclined surface 82 hooks under and against a mating inclined undercut surface 84 in the hook opening 60 when the lid is closed. Hook 58 is attached to lid 50 at a region of relatively thinner material 86 that provides flexibility and resilience to allow the enlarged portion of the hook to pass under and engage the undercut surface 84 of the opening 60

Figure 5F is an enlarged cross-sectional view, taken along line F-F of Fig. 5A, showing the lid latching engagement between at least one of the hooks 58 located on lid 50 and one of the hook openings 60 located in flange 48. This figure is taken at a viewing angle at 90 degrees relative to the viewing angle shown in Figure 5E.

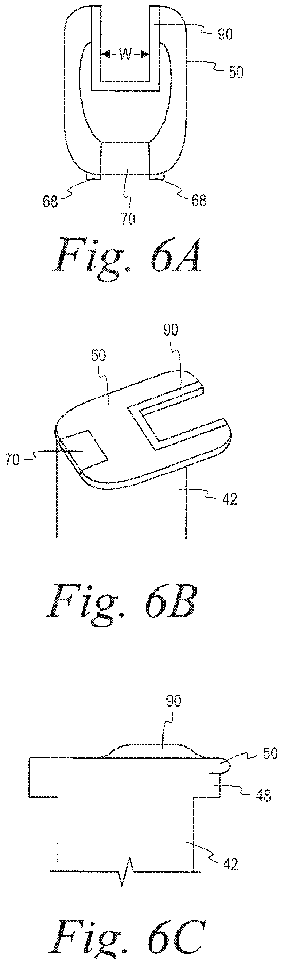

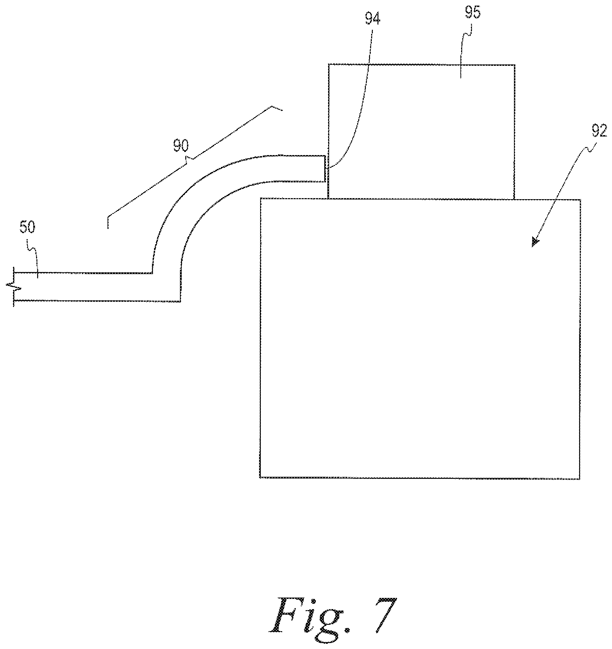

Figs. 6A-6C and Fig. 7 illustrate a modification that may optionally be made to the lid 50 to allow the sample tube holder to accommodate needle assemblies of greater length. More specifically, the lid 50 has a region 90 that extends along at least part of the edge or perimeter of lid aperture 62 and is elevated or raised relative to the plane of the remainder of the lid or the plane of the open end of the body 42, against which the lid lies when closed. As can be seen in Fig. 10B, the raised region may extend fully along the entire inner or peripheral edge of the aperture 62, although that may not be necessary in all embodiments.

Referring to Fig. 7, a portion of the sample tube holder lid 50 with a raised region 90 is shown in an enlarged view. Also, generally shown there is a diagrammatic example of a needle assembly 92 captured within the sample tube holder when the lid is closed. As can be seen there, the illustrated lid 50 has a portion 90 that extends upwardly from the plane of the remainder of the lid. As illustrated, the raised portion extends in a upward arc or curve, terminating at the edge 94 of the lid aperture, although other upwardly extending shapes may be used for the raised region. This raised portion creates a larger (longer) internal space within the sample tube holder between the closed end of the body 42 and the underside of lid 50 when the lid is closed so as to accommodate larger needle assemblies such as the needle assembly 92, which is illustrated as having a length that extends above the plane of most of the lid and the plane of the open end of the body. While accommodating such larger needle assemblies, the lid illustrated in Fig. 7 functions like the lid described earlier, i.e., to allow a proximal portion 94 of the needle assembly 92 to extend through the lid aperture while capturing or confining the remainder 96 of the needle assembly, including the access needle and elongated needle protector portion, within the body 42 of the sample tube holder to further protect against needle sticks.

Figs. 8-11 illustrate sequential steps of using a transparent sample tube holder as described above. A transparent sample tube holder allows user visualization of the steps. Fig. 8 shows the sample tube holder 40 in a position with the lid 50 open and ready for insertion of the used vascular needle assembly 18. An interior sampling needle 98 (located within dust cover 100) may be seen within the body 42 of the sample tube holder. Fig. 9 illustrates a used needle assembly that is attached to tubing 102 and part of a medical fluid flow system 104 that includes the sample tube holder 40 and may include additional components such as containers, filters, Y-junctions, additional tubing, etc. The needle assembly (see 36) is being inserted into the open end of the sample tube holder body 42, which is preferably carried out by gripping the proximal end of the needle assembly, with fingers away from the access needle. The needle assembly may be inserted into the body such as alongside or adjacent to or next to the sampling needle, and if desired the needle assembly may include a visual and/or tactile indicator, such as a colored indicator and/or raised bump or rib, to orient the needle assembly relative to the lid 50 and the aperture 62. Fig. 11 shows the resulting arrangement, with the access needle 20, which cannot be seen because it is covered by the needle protector portion 36, inserted inside the sample tube holder body 42 beside the sampling needle 98, with the lid 50 closed and latched, capturing and confining the access needle and needle protector portion within the sample tube holder body. This serves to enhance protection against accidental needle sticks by either the access needle or the sampling needle and helps confine any blood spillage from the access needle or the sampling needle.

The particular features of this sample tube holder are shown for purposes of illustration and not limitation, and may be varied without departing from this disclosure.

## Claims

1. A sample tube holder (40) including a body (42), open at one end and including a lid (50) pivotally attached to the body and movable to a closed position at least partially closing the open end, the lid (50) including an aperture (62) configured to capture an access needle and needle protector within the body when the lid (50) is in the closed position and a latch to retain the lid (50) in a closed position.

2. The sample tube holder of claim 1 in which the aperture extends through at least one edge of the lid and is sized to capture an access needle and needle protector within the body.

3. The sample tube holder (40) of claim 2 in which the lid (50) is pivotally attached to the body at one side edge and the aperture (62) extends through a side edge opposite the one side edge.

4. The sample tube holder of any one of claims 1 through 3 in which the access needle and needle protector are part of a needle assembly and the aperture is configured to allow a portion of the needle assembly to extend through the aperture while capturing the access needle and needle protector within the body.

5. The sample tube holder (40) of any one of claims 1 through 4 in which a portion of lid (50) bordering the aperture is raised above the plane of the body open end.

6. The sample tube holder (40) of any one of claims 1 through 5 in which the lid (50) is pivotally attached to the body by a hinge (52) comprising a connecting hinge portion and an active hinge portion.

7. The sample tube holder of any one of claims 1 through 6 wherein said lid (50) includes an underside, said tube holder further comprising a flange (48) at the open end and one of the flange (48) and the lid (50) including a latching member and the other of the flange and the lid include a latching member engagement.

8. The sample tube holder of claim 7 in which the latching member comprises a hook (58) projecting from said underside and the latching member engagement comprises a hook receiver (60) in said flange.

9. The sample tube holder of claim 7 wherein the lid (50) comprises at least two latching members (58) projecting from said underside and the flange comprises at least two latching member engagements (60).

10. The sample tube holder of any one of claims 7 through 9 further comprising one or more arcuate segments projecting from said lid underside.

11. A medical fluid flow system including an access needle (20) for accessing the vascular system of a human subject, a needle protector (34) for protecting the access needle from accidental stick after usage, and a sample tube holder (40) including a body (42), open at one end and including a lid (50) pivotally attached to the body (42) and movable to a closed position at least partially closing the open end, a fluid port (64) at an end of the body opposite the open end, the port being configured to allow fluid flow connection with the medical fluid flow system, the lid (50) including an aperture (62) configured to capture an access needle (20) and needle protector (34) within the body when the lid (50) is in the closed position and a latch (58) to retain the lid (50) in a closed position.

12. The medical flow system of claim 11 in which the aperture (62) extends through at least one edge of the lid (50) and is sized to capture an access needle and needle protector within the body.

13. The medical fluid flow system of any one of claims 11 through 12 in which the lid (50) is pivotally attached to the body (42) at one side edge and the aperture (62) extends through a side edge opposite the one side edge.

14. The medical fluid flow system of any one of claims 11 through 13 in which the access needle (20) and needle protector (34) are part of a needle assembly and the aperture (62) is configured to a allow a portion of the needle assembly to extend through the aperture while capturing the access needle (20) and needle protector (34) within the body.

15. The medical fluid flow system of claim 14 in which a portion of lid (50) bordering the aperture (62) is raised above the plane of the body open end.

16. The medical fluid flow system of any one of claims claim 11 through 15 in which the lid (50) is pivotally attached to the body by a hinge (52) comprising a passive hinge portion and an active hinge portion.

17. The medical fluid flow system of claim 11 through 16 comprising a flange (48) at the open end and one of the flange (48) and the lid (50) including a latching member and the other of the flange and the lid include a latching member engagement.

18. The medical fluid flow system of claim 17 wherein said lid (50) includes an underside and the latching member comprises a hook projecting from said lid underside and the engagement comprises a hook receiver in said flange.

19. The medical fluid flow system of claim 17 wherein the lid (50) comprises at least two latching members (58) and the flange (48) comprises at least two latching member engagements (60).

20. The medical fluid flow system of any one of Claims 11 through 19 wherein said fluid port comprises an attachable luer fitting.

## Patentansprüche

1. Probenröhrchenhalter (40), umfassend einen Körper (42), der an einem Ende offen ist und einen Deckel (50) umfasst, der schwenkbar an dem Körper angebracht und in eine geschlossene Position beweglich ist, die das offene Ende mindestens teilweise verschließt, wobei der Deckel (50) eine Öffnung (62) umfasst, die dazu ausgelegt ist, eine Zugangsnadel und einen Nadelschutz in dem Körper aufzunehmen, wenn der Deckel (50) in der geschlossenen Position ist, und eine Verriegelung, um den Deckel (50) in einer geschlossenen Position zu halten.

2. Probenröhrchenhalter nach Anspruch 1, wobei sich die Öffnung durch mindestens einen Rand des Deckels erstreckt und dazu bemessen ist, eine Zugangsnadel und einen Nadelschutz in dem Körper aufzunehmen.

3. Probenröhrchenhalter (40) nach Anspruch 2, wobei der Deckel (50) schwenkbar an einem Seitenrand an dem Körper angebracht ist und sich die Öffnung (62) durch einen Seitenrand gegenüber des einen Seitenrandes erstreckt.

4. Probenröhrchenhalter nach einem der Ansprüche 1 bis 3, wobei die Zugangsnadel und der Nadelschutz Teil einer Nadelanordnung sind und die Öffnung dazu ausgelegt ist, zu ermöglichen, dass sich ein Abschnitt der Nadelanordnung durch die Öffnung erstreckt, während die Zugangsnadel und der Nadelschutz in dem Körper aufgenommen werden.

5. Probenröhrchenhalter (40) nach einem der Ansprüche 1 bis 4, wobei ein Abschnitt des Deckels (50), der an die Öffnung grenzt, über die Fläche des offenen Endes des Körpers angehoben ist.

6. Probenröhrchenhalter (40) nach einem der Ansprüche 1 bis 5, wobei der Deckel (50) schwenkbar durch ein Gelenk (52) an dem Körper angebracht ist, das einen Verbindungsgelenkabschnitt und einen aktiven Gelenkabschnitt umfasst.

7. Probenröhrchenhalter nach einem der Ansprüche 1 bis 6, wobei der Deckel (50) eine Unterseite umfasst, wobei der Röhrchenhalter ferner einen Flansch (48) an dem offenen Ende umfasst und einer von dem Flansch (48) und dem Deckel (50) ein Verriegelungselement umfasst und der andere von dem Flansch und dem Deckel eine Verriegelungselementaufnahme umfasst.

8. Probenröhrchenhalter nach Anspruch 7, wobei das Verriegelungselement einen Haken (58) umfasst, der von der Unterseite vorspringt, und die Verriegelungselementaufnahme einen Hakenaufnehmer (60) in dem Flansch umfasst.

9. Probenröhrchenhalter nach Anspruch 7, wobei der Deckel (50) mindestens zwei Verriegelungselemente (58) umfasst, die von der Unterseite vorspringen, und der Flansch mindestens zwei Verriegelungselementaufnahmen (60) umfasst.

10. Probenröhrchenhalter nach einem der Ansprüche 7 bis 9, ferner umfassend ein oder mehrere bogenförmige Segmente, die von der Deckelunterseite vorspringen.

11. Medizinisches Fluidströmungssystem, umfassend eine Zugangsnadel (20) für den Zugang zum Gefäßsystem eines menschlichen Subjekts, einen Nadelschutz (34) zum Schützen der Zugangsnadel vor versehentlichen Stichen nach der Verwendung, und einen Probenröhrchenhalter (40), der einen Körper (42) umfasst, der an einem Ende offen ist und einen Deckel (50) umfasst, der schwenkbar an dem Körper (42) angebracht und in eine geschlossene Position beweglich ist und das offene Ende mindestens teilweise verschließt, eine Fluidöffnung (64) an einem Ende des Körpers gegenüber dem offenen Ende, wobei die Öffnung dazu ausgelegt ist, eine Fluidströmungsverbindung mit dem medizinischen Fluidströmungssystem zu ermöglichen, wobei der Deckel (50) eine Öffnung (62) umfasst, die dazu ausgelegt ist, eine Zugangsnadel (20) und einen Nadelschutz (34) in den Körper aufzunehmen, wenn der Deckel (50) in der geschlossenen Position ist, und eine Verriegelung (58), um den Deckel (50) in einer geschlossenen Position zu halten.

12. Medizinisches Fluidströmungssystem nach Anspruch 11, wobei sich die Öffnung (62) durch mindestens einen Rand des Deckels (50) erstreckt und dazu bemessen ist, eine Zugangsnadel und einen Nadelschutz in dem Körper aufzunehmen.

13. Medizinisches Fluidströmungssystem nach Anspruch 11 bis 12, wobei der Deckel (50) schwenkbar an einem Seitenrand an dem Körper (42) angebracht ist und sich die Öffnung (62) durch einen Seitenrand gegenüber dem einen Seitenrand erstreckt.

14. Medizinisches Fluidströmungssystem nach einem der Ansprüche 11 bis 13, wobei die Zugangsnadel (20) und der Nadelschutz (34) Teil einer Nadelanordnung sind und die Öffnung (62) dazu ausgelegt ist, zu ermöglichen, dass sich ein Abschnitt der Nadelanordnung durch die Öffnung erstreckt, während die Zugangsnadel (20) und der Nadelschutz (34) in dem Körper aufgenommen werden.

15. Medizinisches Fluidströmungssystem nach Anspruch 14, wobei ein Abschnitt des Deckels (50), der an die Öffnung (62) grenzt, über die Fläche des offenen Endes des Körpers angehoben ist.

16. Medizinisches Fluidströmungssystem nach einem der Ansprüche 11 bis 15, wobei der Deckel (50) durch ein Gelenk (52) schwenkbar an dem Körper angebracht ist, das einen passiven Gelenkabschnitt und einen aktiven Gelenkabschnitt umfasst.

17. Medizinisches Fluidströmungssystem nach einem der Ansprüche 11 bis 16, umfassend einen Flansch (48) an dem offenen Ende, und wobei einer von dem Flansch (48) und dem Deckel (50) ein Verriegelungselement umfasst und der andere von dem Flansch und dem Deckel eine Verriegelungselementaufnahme umfasst.

18. Medizinisches Fluidströmungssystem nach Anspruch 17, wobei der Deckel (50) eine Unterseite umfasst und das Verriegelungselement einen Haken umfasst, der von der Deckelunterseite vorspringt, und die Aufnahme einen Hakenaufnehmer in dem Flansch umfasst.

19. Medizinisches Fluidströmungssystem nach Anspruch 17, wobei der Deckel (50) mindestens zwei Verriegelungselemente (58) umfasst und der Flansch (48) mindestens zwei Verriegelungselementaufnahmen (60) umfasst.

20. Medizinisches Fluidströmungssystem nach einem der Ansprüche 11 bis 19, wobei die Fluidöffnung einen anbringbaren Luer-Ansatz umfasst.

## Revendications

1. Support de tube d'échantillon (40) incluant un corps (42), ouvert à une extrémité et incluant un couvercle (50) attaché de façon pivotante au corps et mobile jusqu'à une position fermée fermant au moins partiellement l'extrémité ouverte, le couvercle (50) incluant une ouverture (62) configurée pour capturer une aiguille d'accès et un protecteur d'aiguille à l'intérieur du corps lorsque le couvercle (50) est dans la position fermée et un verrou pour retenir le couvercle (50) dans une position fermée.

2. Support de tube d'échantillon selon la revendication 1, dans lequel l'ouverture s'étend à travers au moins un bord du couvercle et est dimensionnée pour capturer une aiguille d'accès et un protecteur d'aiguille à l'intérieur du corps.

3. Support de tube d'échantillon (40) selon la revendication 2, dans lequel le couvercle (50) est attaché de façon pivotante au corps au niveau d'un bord latéral et l'ouverture (62) s'étend à travers un bord latéral opposé à l'un bord latéral.

4. Support de tube d'échantillon selon l'une quelconque des revendications 1 à 3, dans lequel l'aiguille d'accès et le protecteur d'aiguille font partie d'un ensemble à aiguille et l'ouverture est configurée pour permettre à une portion de l'ensemble à aiguille de s'étendre à travers l'ouverture tout en capturant l'aiguille d'accès et le protecteur d'aiguille à l'intérieur du corps.

5. Support de tube d'échantillon (40) selon l'une quelconque des revendications 1 à 4, dans lequel une portion de couvercle (50) bordant l'ouverture est élevée au-dessus du plan de l'extrémité ouverte de corps.

6. Support de tube d'échantillon (40) selon l'une quelconque des revendications 1 à 5, dans lequel le couvercle (50) est attaché de façon pivotante au corps par une charnière (52) comprenant une portion de charnière de raccordement et une portion de charnière active.

7. Support de tube d'échantillon selon l'une quelconque des revendications 1 à 6, dans lequel ledit couvercle (50) inclut un côté inférieur, ledit porte-tube comprenant en outre une bride (48) à l'extrémité ouverte et un de la bride (48) et du couvercle (50) incluant un élément de verrouillage et l'autre de la bride et du couvercle incluant un organe d'enclenchement d'élément de verrouillage.

8. Support de tube d'échantillon selon la revendication 7, dans lequel l'élément de verrouillage comprend un crochet (58) faisant saillie à partir dudit côté inférieur et l'organe d'enclenchement d'élément de verrouillage comprend un organe de réception de crochet (60) dans ladite bride.

9. Support de tube d'échantillon selon la revendication 7, dans lequel le couvercle (50) comprend au moins deux éléments de verrouillage (58) faisant saillie à partir dudit côté inférieur et la bride comprend au moins deux organes d'enclenchement d'élément de verrouillage (60).

10. Support de tube d'échantillon selon l'une quelconque des revendications 7 à 9, comprenant en outre un ou plusieurs segments arqués faisant saillie à partir dudit côté inférieur de couvercle.

11. Système d'écoulement de fluide médical incluant une aiguille d'accès (20) pour accéder au système vasculaire d'un sujet humain, un protecteur d'aiguille (34) pour protéger l'aiguille d'accès de piqûre accidentelle après l'usage, et un support de tube d'échantillon (40) incluant un corps (42), ouvert à une extrémité et incluant un couvercle (50) attaché de façon pivotante au corps (42) et mobile jusqu'à une position fermée fermant au moins partiellement l'extrémité ouverte, un orifice de fluide (64) à une extrémité du corps opposée à l'extrémité ouverte, l'orifice étant configuré pour permettre un raccordement d'écoulement de fluide avec le système d'écoulement de fluide médical, le couvercle (50) incluant une ouverture (62) configurée pour capturer une aiguille d'accès (20) et un protecteur d'aiguille (34) à l'intérieur du corps lorsque le couvercle (50) est dans la position fermée et un verrou (58) pour retenir le couvercle (50) dans une position fermée.

12. Système d'écoulement médical selon la revendication 11, dans lequel l'ouverture (62) s'étend à travers au moins un bord du couvercle (50) et est dimensionnée pour capturer une aiguille d'accès et un protecteur d'aiguille à l'intérieur du corps.

13. Système d'écoulement de fluide médical selon l'une quelconque des revendications 11 à 12, dans lequel le couvercle (50) est attaché de façon pivotante au corps (42) au niveau d'un bord latéral et l'ouverture (62) s'étend à travers un bord latéral opposé à l'un bord latéral.

14. Système d'écoulement de fluide médical selon l'une quelconque des revendications 11 à 13, dans lequel l'aiguille d'accès (20) et le protecteur d'aiguille (34) font partie d'un ensemble à aiguille et l'ouverture (62) est configuré pour permettre à une portion de l'ensemble à aiguille de s'étendre à travers l'ouverture tout en capturant l'aiguille d'accès (20) et le protecteur d'aiguille (34) à l'intérieur du corps.

15. Système d'écoulement de fluide médical selon la revendication 14, dans lequel une portion de couvercle (50) bordant l'ouverture (62) est élevée au-dessus du plan de l'extrémité ouverte de corps.

16. Système d'écoulement de fluide médical selon l'une quelconque des revendications 11 à 15, dans lequel le couvercle (50) est attaché de façon pivotante au corps par une charnière (52) comprenant une portion de charnière passive et une portion de charnière active.

17. Système d'écoulement de fluide médical selon la revendication 11 à 16, comprenant une bride (48) à l'extrémité ouverte et un de la bride (48) et du couvercle (50) incluant un élément de verrouillage et l'autre de la bride et du couvercle incluant un organe d'enclenchement d'élément de verrouillage.

18. Système d'écoulement de fluide médical selon la revendication 17, dans lequel ledit couvercle (50) inclut un côté inférieur et l'élément de verrouillage comprend un crochet faisant saillie à partir dudit côté inférieur de couvercle et l'organe d'enclenchement comprend un organe de réception de crochet dans ladite bride.

19. Système d'écoulement de fluide médical selon la revendication 17, dans lequel le couvercle (50) comprend au moins deux éléments de verrouillage (58) et la bride (48) comprend au moins deux organes d'enclenchement d'élément de verrouillage (60).

20. Système d'écoulement de fluide médical selon l'une quelconque des revendications 11 à 19, dans lequel ledit orifice de fluide comprend un raccord Luer attachable.
